# EUROPEAN PATENT APPLICATION

(11) **EP 4 424 255 A1**
(43) Date of publication of application: **04.09.2024**
(21) Application number: 22900285.2
(22) Date of filing: 17.11.2022
(51) Int. Cl.: A61B 18/00

(54) **SURGICAL INSTRUMENT**

(30) Priority: 02.12.2021 CN 202111454666
(71) Applicant: Hocermed (Beijing) Medical Technology Co., Ltd., Beijing 100193 (CN)
(72) Inventor: HUANG, Ze Gang, Beijing 100193 (CN)
(74) Representative: Murgitroyd & Company
(86) International application number: PCT/CN2022/132536
(87) International publication number: WO 2023/098480

(57) **Abstract**

A surgical instrument comprises: a handle assembly comprising a main body (10), a first handle (31) fixedly connected to the main body (10), and a second handle (32) movably connected to the main body (10); an end effector (21) connected to the main body (10) of the handle assembly; a first transmission assembly (41), wherein the second handle (32) moves relative to the first handle (31) to drive the first transmission assembly (41), such that the first transmission assembly (41) drives the end effector (21); and an energy switch (50) configured to switch between an excitation position and a release position, wherein the energy switch (50) is configured to enable energy to be transmitted to the end effector (21) when in the excitation position, and to prevent energy from being transmitted to the end effector (21) when in the release position, and the energy switch (50) is disposed on the first handle (31) and located on a side of the first handle (31) opposite to a side thereof facing towards the second handle (32).

## Description

### CROSS-REFERENCE TO RELATED APPLICATION(S)

The present application claims the priority of Chinese Patent Application No.202111454666.0 filed on December 2, 2021, and the disclosure of the above-mentioned Chinese Patent Application is hereby incorporated in its entirety as a part of the present application.

### TECHNICAL FIELD

The embodiments of the present disclosure belong to the field of medical instruments, and particularly relate to a surgical instrument.

### BACKGROUND

With the development of science and technology, more and more surgical instruments such as bipolar surgical instruments, monopolar surgical instruments and ultrasonic scalpels are introduced into surgical and/or medical procedures. The end effector of the bipolar surgical instrument includes two openable electrodes. During the operation, the two electrodes clamp the target tissue and then are closed; subsequently, an electric energy is applied to the two electrodes to sinter and/or burn the target tissue, so as to close and/or cut off the target tissue. The end effector of the monopolar surgical instrument includes a source electrode and a return electrode, and the target tissue can also be sintered and/or burned through a mechanical clamping action of these two electrodes and an electric energy applied to the source electrode, so as to realize closing and/or cutting off the target tissue. The end effector of the ultrasonic scalpel includes an ultrasonic cutter head and an auxiliary cutter head. During the operation, the ultrasonic cutter head and the auxiliary cutter head clamp the target tissue and then are closed; subsequently, an ultrasonic energy is applied to the ultrasonic cutter head, so as to close and/or cut off the target tissue. That is to say, there are a large number of surgical instruments, the operation process of which involves both the opening and closing of the end effector and the application of energy to the end effector.

### SUMMARY

An embodiment of the present disclosure provides a surgical instrument, including: a handle assembly, including a main body, a first handle and a second handle, wherein the first handle is fixedly connected to the main body, and the second handle is movably connected to the main body; an end effector, connected with the main body of the handle assembly; a first transmission assembly, wherein the second handle, the first transmission assembly and the end effector are configured such that the second handle moves relative to the first handle to drive the first transmission assembly to move, and the first transmission assembly drives the end effector to move; and an energy switch, configured to be switchable between an excitation position and a release position, wherein the energy switch is configured to transmit an energy to the end effector when the energy switch is in the excitation position, and the energy switch is configured not to transmit the energy to the end effector when the energy switch is in the release position, and wherein the energy switch is arranged on the first handle and located at an opposite side of a side of the first handle facing towards the second handle.

For example, the surgical instrument further includes a locking assembly configured to be switchable between a locked position and an unlocked position, wherein the locking assembly and the energy switch are configured such that the energy switch is limited in the release position when the locking assembly is in the locked position; and the locking assembly and the energy switch are configured such that the energy switch is switchable between the excitation position and the release position when the locking assembly is in the unlocked position.

For example, the locking assembly is connected with the second handle, and the second handle moves relative to the first handle to drive the locking assembly to be switched between the locked position and the unlocked position.

For example, the second handle, the end effector and the locking assembly are configured such that the second handle moves towards the first handle to drive the locking assembly to move towards the unlocked position and drive the end effector to move towards a closed position through the first transmission assembly, and the second handle moves away from the first handle to drive the locking assembly to move towards the locked position and drive the end effector to move towards an opened position through the first transmission assembly.

For example, the locking assembly includes a limiting rod, a locking rod and a reset element, wherein the limiting rod is configured to be switchable between a limiting position and a non-limiting position; the locking assembly is configured such that when the locking assembly is in the locked position, the limiting rod is in the limiting position and the reset element is in a reset state, and the locking rod is abutted between the energy switch and the limiting rod to limit the energy switch in the release position; and the locking assembly is configured such that when the locking assembly is in the unlocked position, the limiting rod is in the non-limiting position, an end of the locking rod facing towards the energy switch is abutted against the energy switch, and an end of the locking rod facing towards the limiting rod is not abutted against the limiting rod and becomes a free end, which allows the energy switch to be switchable between the excitation position and the release position; wherein under an action of external force, the energy switch moves from the release position to the excitation position and drives the locking rod to move, and the locking rod drives the reset element to generate a deformation which results in an elastic restoring force; and wherein when the external force is removed, the reset element returns to the reset state under an action of the elastic restoring force and drives the locking rod to move, and the locking rod drives the energy switch to move from the excitation position to the release position.

For example, at least a part of the limiting rod is located in the first handle, and is located at a side of the energy switch facing towards the second handle and is spaced apart from the energy switch by an interval; and the locking rod and the reset element are located in the first handle and are located at the interval between the limiting rod and the energy switch, and extending directions of the locking rod and the reset element intersect with the limiting rod and the energy switch, respectively.

For example, the first handle is provided with an accommodating structure; the reset element is connected with the locking rod, sleeved onto a part of the locking rod and accommodated, together with the part of the locking rod, in the accommodating structure; wherein the accommodating structure is provided with an opening for the end of the locking rod facing towards the limiting rod to pass therethrough, and a size of the reset element is larger than that of the opening in a direction perpendicular to an extending direction of the locking rod; and the locking rod is provided with a limiting boss, and the limiting boss is located in the accommodating structure and is closer to the energy switch than the reset element.

For example, the limiting rod is connected with the second handle, and wherein the second handle moves towards the first handle to drive the limiting rod to move towards the non-limiting position, and the second handle moves away from the first handle to drive the limiting rod to move towards the limiting position.

For example, the surgical instrument includes a first sliding slot at least partially arranged inside the main body of the handle assembly, and the first sliding slot has a first end close to the first handle and a second end away from the first handle; a part of the second handle located in the main body of the handle assembly is provided with a second sliding slot, wherein the second sliding slot is arranged at a side of the part of the second handle facing towards the first handle, and wherein the second sliding slot has a first end close to the second handle and a second end away from the second handle; the limiting rod is provided with a first protrusion and a second protrusion which are arranged opposite to each other, wherein the first protrusion is slidably connected in the first sliding slot, and the second protrusion is slidably connected in the second sliding slot; the first sliding slot is cooperated with the first protrusion and the second sliding slot is cooperated with the second protrusion such that, the second handle moves towards the first handle to allow the second protrusion to slide, in the second sliding slot, from the second end of the second sliding slot towards the first end of the second sliding slot, and allow the first protrusion to slide from the first end of the first sliding slot towards the second end of the first sliding slot, so as to drive the limiting rod to the non-limiting position; and the first sliding slot is cooperated with the first protrusion and the second sliding slot is cooperated with the second protrusion such that, the second handle moves away from the first handle to allow the second protrusion to slide, in the second sliding slot, from the first end of the second sliding slot towards the second end of the second sliding slot, and allow the first protrusion to slide from the second end of the first sliding slot towards the first end of the first sliding slot, so as to drive the limiting rod to the limiting position.

For example, an extending direction of the first sliding slot coincides with an extending direction of the limiting rod; and an extending direction of the second sliding slot intersects with the extending direction of the first sliding slot.

For example, the surgical instrument further includes a prompt tone elastic piece arranged inside the first handle, wherein the limiting rod is provided with a third protrusion; and when the second handle moves towards the first handle to drive the limiting rod to the non-limiting position, the third protrusion triggers the prompt tone elastic piece to generate a prompt tone.

For example, the energy switch is provided with an excitation protrusion protruding towards an inside of the first handle; a circuit switch is arranged inside the first handle; and the energy switch is configured such that, when the energy switch is in the excitation position, the excitation protrusion presses the circuit switch to allow the circuit switch to conduct an energy path from an energy source to the end effector, so that an energy is transmitted from the energy source to the end effector; and the energy switch is further configured such that, when the energy switch is in the release position, the excitation protrusion is spaced apart from the circuit switch to prevent the circuit switch from conducting the energy path from the energy source to the end effector, so that the energy cannot be transmitted from the energy source to the end effector.

For example, the energy switch is used as a part of a housing of the first handle.

For example, the energy switch has a first end and a second end opposite to each other along an extending direction of the energy switch, and the second end is closer to the main body of the handle assembly than the first end; in a direction from the first end to the second end of the energy switch, the energy switch includes a first part and a second part, wherein the second part is closer to the main body of the handle assembly than the first part, and wherein the second part is integrally formed with the first part and is bent from the first part towards a direction away from the first handle; at any position on the first part, a section of the first part perpendicular to the extending direction of the energy switch is in an arched shape, and a height of the arched shape increases along a direction from the second end to the first end of the energy switch.

For example, the second part is bent such that an outer contour of an end of the second part close to the main body of the handle assembly matches an outer contour of the main body of the handle assembly.

For example, the energy switch has a first end and a second end opposite to each other along an extending direction of the energy switch, and the second end is closer to the main body of the handle assembly than the first end; an engagement slot is arranged at the second end of the energy switch, and a pivot is arranged inside the first handle, wherein the engagement slot is engaged with the pivot and is rotatable around the pivot; a first engagement structure protruding towards an inside of the first handle is arranged between the first end and the second end of the energy switch, and a second engagement structure is arranged inside the first handle; wherein when the energy switch is in the release position, the first engagement structure and the second engagement structure are engaged with each other; and when the energy switch is switched between the excitation position and the release position, the first engagement structure moves relative to the second engagement structure so as to be away from or close to the second handle.

For example, the first handle includes a first half-shell and a second half-shell which are connected with each other to define a cavity and a groove located outside the cavity; the pivot and the second engagement structure are located inside the cavity, and a bottom of the groove is provided with a first opening and a second opening; wherein the engagement slot is engaged with the pivot through the first opening, and the first engagement structure is engaged with the second engagement structure through the second opening, so that the energy switch is connected to the first handle and covers the groove.

For example, a side of the first handle facing towards the second handle is not provided with a structure which is deformable under an action of pressing.

For example, the surgical instrument includes a reset mechanism; the end effector includes a first arm and a second arm which are openable and closable; and the second handle, the first transmission assembly, the reset mechanism and the end effector are configured such that, under an action of an external force, the second handle moves towards the first handle to drive the first transmission assembly to move, the reset mechanism generates a deformation resulting in an elastic restoring force, and the first transmission assembly drives the first arm and the second arm of the end effector to be closed; and when the external force is removed, the reset mechanism is reset under an action of the elastic restoring force and drives the first transmission assembly to move, thereby driving the second handle to move away from the first handle and driving the first arm and the second arm of the end effector to be opened.

For example, the surgical instrument further includes a blade and a second transmission assembly, wherein the blade is connected to an end of the second transmission assembly facing towards the end effector; and the handle assembly further includes a third handle movably connected to the main body of the handle assembly, and wherein the third handle, the second transmission assembly and the blade are configured such that, the third handle moves relative to the first handle to drive the second transmission assembly to move, and the second transmission assembly drives the blade to move towards or away from the end effector.

### BRIEF DESCRIPTION OF DRAWINGS

In order to explain the technical solution of the embodiments of the present disclosure more clearly, the accompanying drawings of the embodiments will be briefly introduced below. Obviously, the described drawings in the following description only relate to some embodiments of the present disclosure, and are not intended to limit the present disclosure.
Fig. 1 is an overall schematic view of a surgical instrument provided by an embodiment of the present disclosure;
Fig. 2 is a schematic view illustrating an internal structure of part A of the surgical instrument shown in Fig. 1, in which a second handle is in an initial state, an energy switch is in a release position, and a third handle is in an initial state;
Fig. 3 is a schematic view illustrating an internal structure of part A of the surgical instrument shown in Fig. 1, in which a second handle is pressed towards a first handle, an energy switch is in a release position, and a third handle is in an initial state;
Fig. 4 is a schematic view illustrating an internal structure of part A of the surgical instrument shown in Fig. 1, in which a second handle is pressed towards a first handle, an energy switch is in an excitation position, and a third handle is in an initial state;
Fig. 5 is a schematic view illustrating an internal structure of part A of the surgical instrument shown in Fig. 1, in which a second handle is pressed towards a first handle, an energy switch is in an excitation position, and a third handle is in a knife-pushing state;
Fig. 6 is a schematic exploded view of part B of the surgical instrument shown in Fig. 1;
Fig. 7a is an overall schematic view of an energy switch of the surgical instrument shown in Fig. 1;
Fig. 7b is a schematic view illustrating an external structure of part A of the surgical instrument shown in Fig. 1, in which a first handle is not equipped with an energy switch; and
Fig. 8 is another overall schematic view of a surgical instrument provided by an embodiment of the present disclosure.

### DETAILED DESCRIPTION

In order to make objects, technical details and advantages of the embodiments of the present disclosure apparent, the technical solutions of the embodiments will be described in a clearly and fully understandable way in connection with the drawings related to the embodiments of the present disclosure. Apparently, the described embodiments are just a part but not all of the embodiments of the present disclosure. Based on the described embodiments herein, those skilled in the art can obtain other embodiment(s), without any inventive work, which should be within the scope of the present disclosure.

Unless otherwise defined, all the technical and scientific terms used herein have the same meanings as commonly understood by one of ordinary skill in the art to which the present disclosure belongs. The terms "first," "second," etc., which are used in the present disclosure, are not intended to indicate any sequence, amount or importance, but distinguish various components. Also, the terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

The drawings of the present disclosure are not drawn strictly to actual scales, and the specific size and number of each structure can be determined according to actual needs. The drawings described in the present disclosure are only schematic structural views.

It should be noted that although most of the drawings of the present disclosure depict bipolar electrosurgical scalpel used in conjunction with laparoscopic surgery, the embodiments of the present disclosure are not limited to this. The surgical instrument of the embodiments of the present disclosure can also be used for traditional open surgical procedures, and the surgical instrument of the embodiments of the present disclosure can also be a monopolar electrosurgical scalpel, an ultrasonic scalpel, or any surgical instrument involving the opening and closing operations of the end effector and the energy application operation.

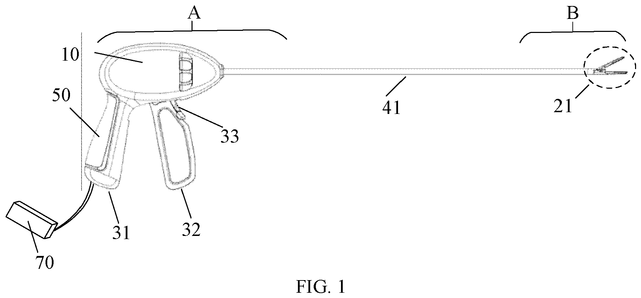

According to an embodiment of the present disclosure, a surgical instrument is provided. Fig. 1 is an overall schematic view of a surgical instrument provided by an embodiment of the present disclosure. Referring to Fig. 1, a surgical instrument according to an embodiment of the present disclosure includes: a handle assembly, including a main body 10, a first handle 31 and a second handle 32, wherein the first handle 31 is fixedly connected to the main body 10, and the second handle 32 is movably connected to the main body 10; an end effector 21, connected with the main body 10 of the handle assembly; a first transmission assembly 41, wherein the second handle 32, the first transmission assembly 41 and the end effector 21 are configured such that: the second handle 32 moves relative to the first handle 31 to drive the first transmission assembly 41 to move, and the first transmission assembly 41 drives the end effector 21 to move; and an energy switch 50, configured to be switchable between an excitation position and a release position, wherein the energy switch 50 is configured to transmit an energy to the end effector 21 when the energy switch 50 is in the excitation position, and is configured not to transmit the energy to the end effector 21 when the energy switch 50 is in the release position, and wherein the energy switch 50 is arranged at the first handle 31 and located at an opposite side of a side of the first handle 31 facing towards the second handle 32.

According to the embodiment of the present disclosure, the energy switch 50 is located at an opposite side of a side of the first handle 31 facing towards the second handle 32; that is, the energy switch 50 and the second handle 32 are located at two opposite sides of the first handle 31, respectively. When the surgical instrument is operated, an operator holds the first handle 31 with a single hand in a grasping posture; by arranging the energy switch 50 and the second handle 32 at two opposite sides of the first handle 31, respectively, the operator can manipulate the second handle 32 by pressing the second handle 32 towards the first handle 31 with the finger(s) of the single hand, and manipulate the energy switch 50 by pressing the energy switch 50 towards the first handle 31 with the palm of the single hand. In this way, the movement of the second handle 32 and the energy switch 50 moving towards each other in opposite directions matches the movement of the fingers and the palm, when the single hand is in a grasping posture, moving towards each other in opposite directions, so that the operation process of the surgical instrument matches the operator's grasping posture with a single hand in a better way, which is more ergonomic and more convenient for operation. Further, according to the embodiment of the present disclosure, the energy switch 50 is arranged at the first handle 31, which on the one hand enables the structure of the surgical instrument to be more compact and simpler, and on the other hand is more convenient for the palm of the hand holding the first handle 31 to press and control the energy switch 50, thus increasing the convenience of operation.

It should be noted that since the first handle 31 is fixedly connected with the main body 10 of the handle assembly, the second handle 32 and a third handle 33 described below move relative to the first handle 31, that is, relative to the main body 10 of the handle assembly.

For example, according to an embodiment of the present disclosure, the first transmission assembly 41 is connected between the main body 10 of the handle assembly and the end effector 21.

For example, according to an embodiment of the present disclosure, the first handle 31 is farther from the end effector than the second handle 32.

For example, when the surgical instrument according to the embodiment of the present disclosure is operated, the second handle 32 moves towards the first handle 31 to drive the end effector 21 to be closed through the first transmission assembly 41; after the end effector 21 is closed, the energy switch 50 is pressed to reach the excitation position, so that an energy is transmitted to the end effector 21; that is to say, the operation of pressing the second handle 32 towards the first handle 31 by finger(s) is prior to the operation of pressing the energy switch 50 towards the first handle 31 by the palm. In order to prevent the palm from undesirably pressing the energy switch 50 to the excitation position in advance during the operation of pressing the second handle 32 towards the first handle 31 by finger(s), the surgical instrument according to an embodiment of the present disclosure further includes a locking assembly. For example, the locking assembly is configured to be switchable between a locked position and an unlocked position; the locking assembly and the energy switch 50 are configured such that the energy switch 50 is limited in the release position when the locking assembly is in the locked position; the locking assembly and the energy switch 50 are configured such that the energy switch 50 is switchable between the excitation position and the release position when the locking assembly is in the unlocked position. The locking assembly locks the energy switch 50; and the locking assembly unlocks the energy switch 50 only when it is necessary to press the energy switch 50 to the excitation position. In this way, it can prevent the palm from undesirably pressing the energy switch 50 to the excitation position in advance during the operation of pressing the second handle 32 towards the first handle 31 by finger(s), and can avoid any accidental touch with the energy switch 50 which may allow the energy switch 50 to reach the excitation position undesirably.

For example, according to the embodiment of the present disclosure, the locking assembly is connected with the second handle 32, and the second handle 32 moves relative to the first handle 31 to drive the locking assembly to be switched between the locked position and the unlocked position. With this arrangement, the locking assembly can be controlled by the second handle 32, and no specialized control element is necessary for the locking assembly, which enables the structure of the surgical instrument to be more compact and simpler. Further, the second handle 32 moves towards the first handle 31 to drive the locking assembly to move towards the unlocked position, and the second handle 32 moves away from the first handle 31 to drive the locking assembly to move towards the locked position; in addition, the movement of the second handle 32 towards the first handle 31 can also drive the end effector 21 to move towards a closed position through the first transmission assembly 41, and the movement of the second handle 32 away from the first handle 31 can also drive the end effector 21 to move towards an opened position through the first transmission assembly 41. In this way, by manipulating the second handle 32, the unlocking process of the locking assembly is synchronized with the closing process of the end effector 21, and the locking process of the locking assembly is synchronized with the opening process of the end effector 21, which simplifies the operation steps and simplifies the structure of the surgical instrument according to the embodiment of the present disclosure. For example, once the second handle 32 that moves towards the first handle 31 arrives at a position where the end effector 21 is closed, the locking assembly will also reach the unlocked position along with the second handle 32, thus releasing the locking effect on the energy switch 50 applied by the locking assembly; that is to say, the energy switch 50 is unlocked at the same time when the end effector 21 is closed, so that the energy application operation of the energy switch 50 can be smoothly performed immediately after the closing operation of the end effector 20.

Referring to Fig. 1, the surgical instrument according to the embodiment of the present disclosure is connected to an external energy source 70 through a conductive wire; however, the embodiment of the present disclosure is not limited to this, and the surgical instrument according to the embodiment of the present disclosure may also adopt a built-in energy source.

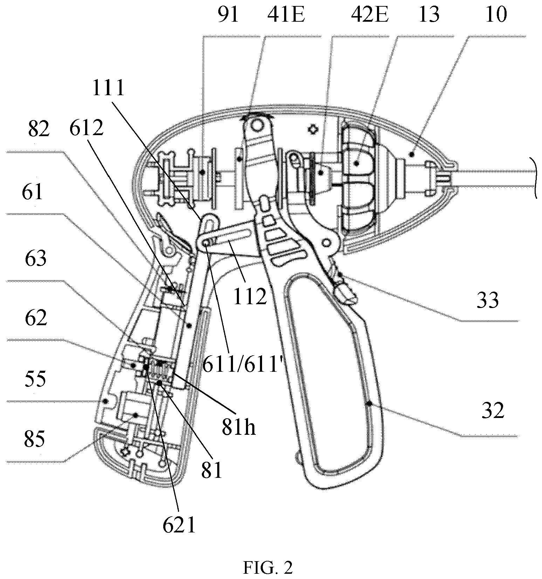

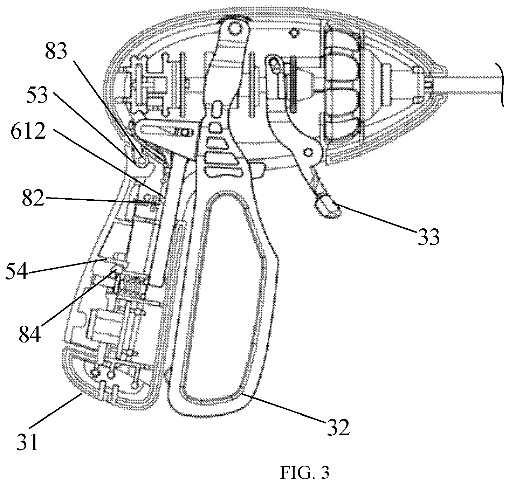

For the convenience of subsequent description, part A and part B of the surgical instrument are marked in Fig. 1; Figs. 2 to 5 are schematic views of an internal structure of the part A. In Figs. 2 to 5, the second handle 32, the energy switch 50 and the third handle 33 have different positions and states. Fig. 2 shows an initial state of the surgical instrument, in which the second handle 32 is away from the first handle 31 and is in an initial state where it has not been pressed yet, the energy switch 50 is in the release position, and the third handle 33 is in an initial state. In Fig. 3, the second handle 32 is pressed towards the first handle 31, the energy switch 50 is in the release position, and the third handle 33 is in an initial state. Referring to Figs. 2 and 3, the locking assembly includes a limiting rod 61, a locking rod 62 and a reset element 63, wherein the limiting rod 62 is configured to be switchable between a limiting position and a non-limiting position; referring to Fig. 2, the locking assembly is configured such that when it's in the locked position, the limiting rod 61 is in the limiting position and the reset element 63 is in a reset state, and the locking rod 62 is abutted between the energy switch 50 and the limiting rod 61 to limit the energy switch 50 in the release position; referring to Fig. 3, the locking assembly is configured such that when it's in the unlocked position, the limiting rod 61 is in the non-limiting position, the end of the locking rod 62 facing towards the energy switch 50 is abutted against the energy switch 50, and the end of the locking rod 62 facing towards the limiting rod 61 is not abutted against the limiting rod 61 and becomes a free end, so that the energy switch 50 is switchable between the excitation position and the release position. For example, under the action of an external force (that is, when pressed by the palm), the energy switch 50 moves from the release position to the excitation position and drives the locking rod 62 to move, and the locking rod 62 drives the reset element 63 to generate a deformation which results in an elastic restoring force; when the external force is removed (that is, the energy switch 50 is no longer pressed by the palm), the reset element 63 returns to the reset state under the action of the elastic restoring force and drives the locking rod 62 to move, and the locking rod 62 drives the energy switch 50 to move from the excitation position to the release position. By providing the locking assembly with the above-described structure, not only can the energy switch 50 be locked, but also can the energy switch 50 be reset to the release position after the energy is applied.

For example, referring to Figs. 2 and 3, at least a part of the limiting rod 61 is located inside the first handle 31, and is located at the side of the energy switch 50 facing towards the second handle 32 and spaced apart from the energy switch 50 by an interval; the locking rod 62 and the reset element 63 are located inside the first handle 31 and located at the interval between the limiting rod 61 and the energy switch 50, and extending directions of the locking rod 62 and the reset element 63 intersect with the limiting rod 61 and the energy switch 50, respectively. Further, for example, the limiting rod 61 and the energy switch 50 extend generally along an extending direction of the first handle 31, and the locking rod 62 generally perpendicularly intersects with the limiting rod 61 and the energy switch 50, respectively.

For example, with continued reference to Figs. 2 and 3, the first handle 31 is internally provided with an accommodating structure 81; the reset element 63 is connected with the locking rod 62, sleeved onto a part of the locking rod 62, and accommodated, together with the part of the locking rod 62, in the accommodating structure 81. The accommodating structure 81 has an opening 81h for the end of the locking rod 62 facing towards the limiting rod 61 to pass therethrough, and the size of the reset element 63 is larger than the size of the opening 81h in the direction perpendicular to the extending direction of the locking rod 62. With such arrangement, the reset element 63 can be confined in the accommodating structure 81, so that when the energy switch 50 is pressed by the palm, the locking rod 62 can drive the reset element 63 to generate a deformation which results in an elastic restoring force. For example, with continued reference to Figs. 2 and 3, the locking rod 62 has a limiting boss 621, which is located in the accommodating structure 81 and is closer to the energy switch 50 than the reset element 63. As mentioned above, when the external force is removed (that is, the energy switch 50 is no longer pressed by the palm), the reset element 63 returns to the reset state under the action of the elastic restoring force and drives the locking rod 62 to move, and the locking rod 62 drives the energy switch 50 to move from the excitation position to the release position; in this case, in order to prevent the energy switch 50 from being bounced off and separated from the first handle 31 under the action of the elastic restoring force of the reset element 63, the above-mentioned limiting boss 621 is provided. Through the cooperation between the reset element 63 and the limiting boss 621, the energy switch 50 can be accurately reset to the release position after the external force is removed.

As described above, the locking assembly is connected with the second handle 32. For example, more specifically, referring to Figs. 2 and 3, the limiting rod 61 of the locking assembly is connected with the second handle 32, the second handle 32 moves towards the first handle 31 to drive the limiting rod 61 to the non-limiting position, and the second handle 32 moves away from the first handle 31 to drive the limiting rod 61 to the limiting position. For example, the following structure is adopted to allow the limiting rod 61 to move along with the second handle 32. With continued reference to Figs. 2 and 3, the surgical instrument includes a first sliding slot 111 at least partially arranged inside the main body 10 of the handle assembly, and the first sliding slot 111 has a first end close to the first handle 31 and a second end away from the first handle 31. A part of the second handle 32 located in the main body 10 of the handle assembly is provided with a second sliding slot 112 which is arranged at the side of the part of the second handle 32 facing towards the first handle 31, and the second sliding slot 112 has a first end close to the second handle 32 and a second end away from the second handle 32. The limiting rod 61 has a first protrusion 611 and a second protrusion 611' which are arranged opposite to each other, the first protrusion 611 is slidably connected in the first sliding slot 111 and the second protrusion 611' is slidably connected in the second sliding slot 112. The first sliding slot 111 is cooperated with the first protrusion 611 and the second sliding slot 112 is cooperated with the second protrusion 611' such that: the second handle 32 moves towards the first handle 31 to allow the second protrusion 611' to slide, in the second sliding slot 112, from the second end of the second sliding slot 112 towards the first end of the second sliding slot 112, and allow the first protrusion 611 to slide from the first end of the first sliding slot 111 towards the second end of the first sliding slot 111, so as to drive the limiting rod to the non-limiting position. The first sliding slot 111 is cooperated with the first protrusion 611 and the second sliding slot 112 is cooperated with the second protrusion 611' such that: the second handle 32 moves away from the first handle 31 to allow the second protrusion 611' to slide, in the second sliding slot 112, from the first end of the second sliding slot 112 towards the second end of the second sliding slot 112, and allow the first protrusion 611 to slide from the second end of the first sliding slot 111 towards the first end of the first sliding slot 111, so as to drive the limiting rod to the limiting position. For example, the first sliding slot 111 is entirely located in the main body 10 of the handle assembly. For example, a part of the first sliding slot 111 is located in the main body 10 of the handle assembly, and the other part of the first sliding slot 111 is located in the first handle 31. For example, the extending direction of the first sliding slot 111 coincides with the extending direction of the limiting rod 61; the extending direction of the second sliding slot 112 intersects with the extending direction of the first sliding slot 111, so that the second handle 21 drives the limiting rod 61 to move more smoothly. For example, referring to Fig. 2, the second handle 32 has a protrusion located in the main body 10 of the handle assembly, which protrusion is protruding from the second handle 32 towards the first handle 31; and the above-described second sliding slot 112 is provided at the protrusion. By manipulating the second handle 32, the limiting rod 61 reaches the non-limiting position at the same time when the end effector 21 is closed, which allows the locking assembly to reach the unlocked position and allows the energy switch 50 to be unlocked.

For example, with continued reference to Fig. 2 and Fig. 3, the surgical instrument according to the embodiment of the present disclosure further includes a prompt tone elastic piece 82 arranged inside the first handle 31; the limiting rod 61 is provided with a third protrusion 612; when the second handle 32 moves towards the first handle 31 to drive the limiting rod 61 to the non-limiting position, the third protrusion 612 triggers the prompt tone elastic piece 82 to generate a prompt tone. When the limiting rod 61 reaches the non-limiting position, it indicates that the locking assembly reaches the unlocked position, the locking effect on the energy switch 50 applied by the locking assembly is released, and the end effector 21 is closed at the same time. By providing the prompt tone elastic piece 82 and the third protrusion 612 which are cooperated with each other, and by enabling the third protrusion 612 to trigger the prompt tone elastic piece 82 to produce a prompt tone when the limiting rod 61 reaches the non-limiting position, the operator can be reminded that the end effector 21 has been closed and the energy switch 50 has been unlocked, and then the operator can safely carry out the subsequent operation of energy application. As can be seen from Fig. 2 in combination with Fig. 3, the third protrusion 612 moves, along with the limiting rod 61, to collide with the prompt tone elastic piece 82, and the prompt tone elastic piece 82 produces the prompt tone upon collision.

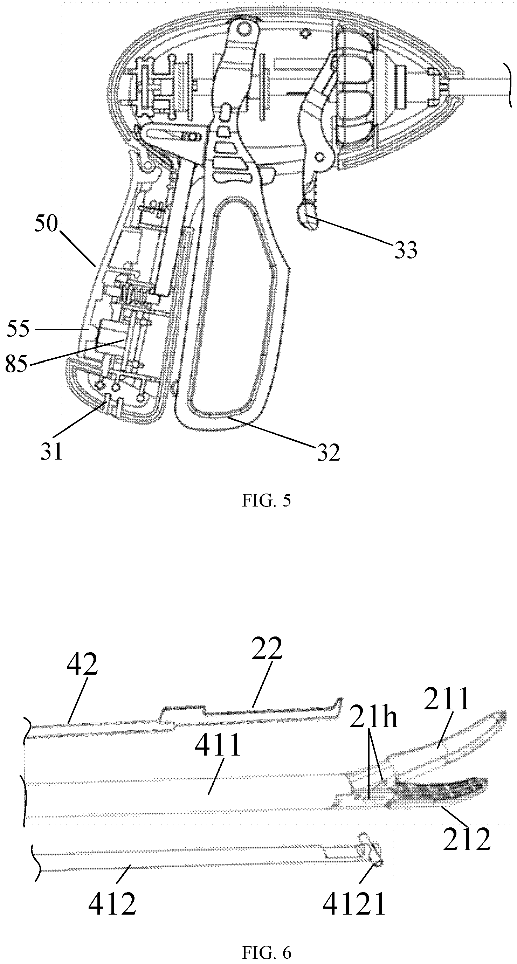

Fig. 6 is a schematic exploded view of part B of the surgical instrument shown in Fig. 1. It should be noted that in the surgical instrument according to the embodiment of the present disclosure, a pulling rod 412, a blade 22 and a second transmission assembly 42 connected therewith shown in Fig. 6 are all housed in a sleeve 411; for convenience of understanding, the pulling rod 412, the blade 22 and the second transmission assembly 42 are shown in an exploded state in Fig. 6. Referring to Fig. 1 in combination with Fig. 6, the first transmission assembly 41 includes a sleeve 411 and a pulling rod 412; the end effector 21 includes a first arm 211 and a second arm 212 which are openable and closable, wherein one of the first and second arms 211, 212 is fixedly connected to the sleeve 411, and the other one is rotatably connected to the sleeve 411; each of the first arm 211 and the second arm 212 has a pin hole 21h, and the end of the pulling rod 412 has a pin 4121 fitted with the pin hole 21h. After inserting the pin 4121 into the pin hole 21h of each of the first arm 211 and the second arm 212, the operator presses the second handle 32 towards the first handle 31 to cause a relative movement between the sleeve 411 and the pulling rod 412, which drives the pin 4121 to move in the pin hole 21h so as to open and close the first arm 211 and the second arm 212. For example, the second handle 32 moves towards the first handle 31 to drive the end 41E of the first transmission assembly 41, so that a relative movement occurs between the sleeve 411 and the pulling rod 412. For example, when the first arm 211 and the second arm 212 are opened, the end effector 21 is opened; when the first arm 211 and the second arm 212 are closed, the end effector 21 is closed.

Referring to Fig. 2, the surgical instrument according to the embodiment of the present disclosure further includes a reset mechanism 91; the second handle 32, the first transmission assembly 41, the reset mechanism 91 and the end effector 21 are configured such that: under the action of an external force (that is, when pressed by the operator's finger), the second handle 32 moves towards the first handle 31 to drive the first transmission assembly 41 to move, the reset mechanism 91 generates a deformation which results in an elastic restoring force, and the first transmission assembly 41 drives the first arm 211 and the second arm 212 of the end effector 21 to be closed; and when the external force is removed (that is, the second handle 32 is no longer pressed by the operator's finger), the reset mechanism resets under the action of the elastic restoring force and drives the first transmission assembly 41 to move, thereby driving the second handle 32 to move away from the first handle 31 and driving the first arm 211 and the second arm 212 of the end effector 21 to be opened.

For example, referring to Figs. 1 and 6, the surgical instrument according to the embodiment of the present disclosure further includes a blade 22 and a second transmission assembly 42, and the blade 22 is connected to the end of the second transmission assembly 42 facing towards the end effector 21; the handle assembly further includes a third handle 33 movably connected to the main body 10 of the handle assembly. The third handle 33, the second transmission assembly 42 and the blade 22 are configured such that: the third handle 33 moves relative to the first handle 31 to drive the second transmission assembly 42 to move, and the second transmission assembly 42 drives the blade 22 to move towards or away from the end effector 21. For example, the operator's finger presses the third handle 33 to move towards the first handle 31, the third handle 33 pushes the end 42E of the second transmission assembly 42 (see Fig. 2) to move, and the second transmission assembly 42 pushes the blade 22 towards the end of the end effector 21 away from the main body 10 of the handle assembly so as to cut the tissue clamped by the end effector 21. When the external force is removed (that is, the fingers of the operator no longer press the third handle 33), the third handle 33 and the second transmission assembly 42 return to the initial state, and the blade 22 moves in a direction away from the end effector 21.

For example, according to an embodiment of the present disclosure, the second transmission assembly 42 is connected between the main body 10 of the handle assembly and the blade 22.

For example, in the embodiment according to the present disclosure, the second handle 32 is farther from the end effector 21 than the third handle 33, and the first handle 31 is farther from the end effector 21 than the second handle 32.

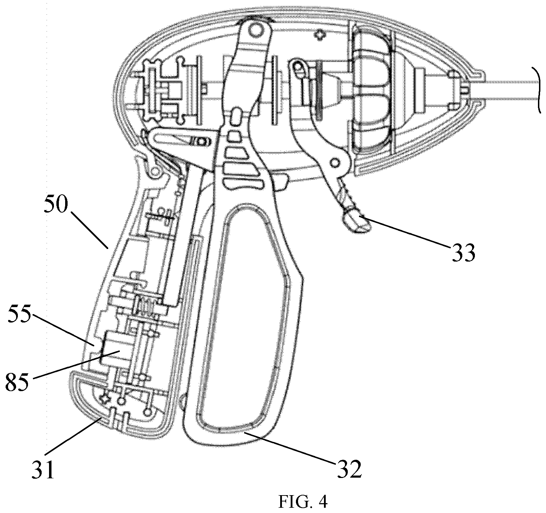

With combined reference to Figs. 1-6, the surgical instrument according to the embodiment of the present disclosure is operated as follows. In the initial state shown in Fig. 2, the second handle is in the initial state, the energy switch is in the release position, the third handle is in the initial state, and the end effector 21 is in the opened state. The operator holds the first handle 31 with a single hand and locates the surgical instrument at a position, where the target tissue is located, between the first arm 211 and the second arm 212 of the end effector 21. Referring to Fig. 3, the operator presses the second handle 32 towards the first handle 31 with finger(s), the first arm 211 and the second arm 212 of the end effector 21 are closed to clamp the target tissue, the limiting rod 61 reaches the non-limiting position to release the energy switch 50 from the locking of the locking assembly, and the third protrusion 612 collides with the prompt tone elastic piece 82 to produce a prompt tone to prompt the operator to perform the next operation. Referring to Fig. 4, the operator presses the energy switch 50 with the palm to allow the energy switch 50 to reach the excitation position, so that an energy is transmitted from the energy source 70 to the end effector 21 to close the target tissue. Referring to Fig. 5, the operator presses the third handle 33 towards the first handle 31 with finger(s) (e.g., fingers different from those manipulating the second handle 32) to push the blade 22 towards the end of the end effector 21 away from the main body 10 of the handle assembly, so as to cut the target tissue. After cutting, the operator releases the second handle 32, the third handle 33 and the energy switch 50; the second handle 32 and the third handle 33 are reset under the action of the first transmission assembly 41 and the second transmission assembly 42; the locking rod 62 is reset under the action of the elastic restoring force of the reset element 63, so as to drive the energy switch 50 to be reset; and the limiting rod 61, driven by the second handle 32, reaches the limiting position to push the locking rod 63 to be abutted between the limiting rod 61 and the energy switch 50, so that the energy switch 50 is locked in the release position, and the whole surgical instrument restores to the initial state as shown in Fig. 2.

For example, referring to Figs. 2 and 4, the energy switch 50 has an excitation protrusion 55 protruding towards the inside of the first handle 31; a circuit switch 85 is arranged inside the first handle 31; the energy switch 50 is configured such that, when the energy switch 50 is in the excitation position, the excitation protrusion 55 presses the circuit switch 85 to allow the circuit switch 85 to conduct an energy path from the energy source 70 to the end effector 21, and allow an energy to be transmitted from the energy source 70 to the end effector 21; the energy switch 50 is configured such that, when the energy switch 50 is in the release position, the excitation protrusion 55 is spaced apart from the circuit switch 85 to prevent the circuit switch 85 from conducting the energy path from the energy source 70 to the end effector 21, and prevent the energy from being transmitted from the energy source 70 to the end effector 21.

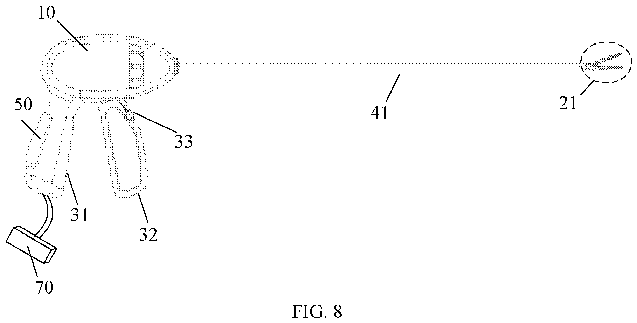

As described above, the energy switch 50 is arranged at the first handle 31; further, for example, the energy switch 50 is used as a part of the housing of the first handle 31, so that the structure of the surgical instrument according to the embodiment of the present disclosure is simpler and more aesthetic. For example, referring to Fig. 2, in the extending direction of the first handle 31, the energy switch 50 basically extends from the end of the first handle 31 close to the main body 10 of the handle assembly to the end of the first handle 31 away from the main body 10 of the handle assembly. Fig. 8 is another overall schematic view of the surgical instrument provided by the embodiment of the present disclosure; for example, referring to Fig. 8, the size of the energy switch 50 can be appropriately reduced compared with that of Fig. 2, so that the energy switch 50 is located in the middle area of the first handle 31 in the extending direction of the first handle 31.

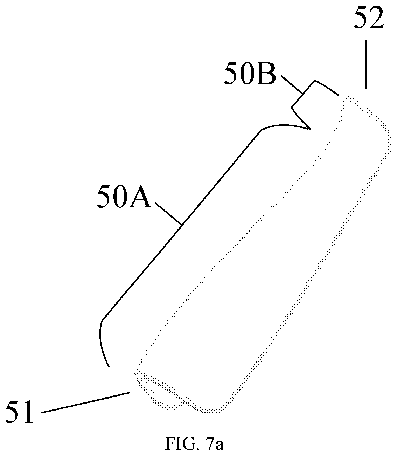

Fig. 7a is an overall schematic view of the energy switch of the surgical instrument shown in Fig. 1. Referring to Fig. 7a, the energy switch 50 has a first end 51 and a second end 52 opposite to each other along an extending direction of the energy switch 50, and the second end 52 is closer to the main body 10 of the handle assembly than the first end 51. In a direction from the first end 51 to the second end 52 of the energy switch 50, the energy switch 50 includes a first part 50A and a second part 50B, the second part 50B is closer to the main body 10 of the handle assembly than the first part 50A, and the second part 50B is integrally formed with the first part 50A and is bent from the first part 50A towards a direction away from the first handle 31. At any position on the first part 50A, the section of the first part 50A perpendicular to the extending direction of the energy switch 50 has an arched shape, and the height of the arched shape increases in the direction from the second end 52 to the first end 51 of the energy switch 50. Through the above design, the contour of the energy switch 50 can be matched with the contour of the first handle 31, the energy switch 50 can have a larger area for the operator to press conveniently, and the increase of the height of the arched shape can facilitate providing a sufficient accommodation space for the locking rod 62, the excitation protrusion 55 and the circuit switch 85. For example, the second part 50B is bent such that its end close to the main body 10 of the handle assembly matches the outer contour of the main body 10 of the handle assembly, which allows the contour of the first handle 31 and the contour of the main body 10 to be smoothly joined with each other.

Referring to Fig. 2, Fig. 3 and Fig. 7a, the energy switch 50 has a first end 51 and a second end 52 opposite to each other along the extending direction of the energy switch 50, and the second end 52 is closer to the main body 10 of the handle assembly than the first end 51. An engagement slot 53 is arranged at the second end 52 of the energy switch, a pivot 83 is arranged inside the first handle 31, and the engagement slot 53 is engaged with the pivot 83 and is rotatable around the pivot 83. A first engagement structure 54 protruding towards the inside of the first handle 31 is arranged between the first end 51 and the second end 52 of the energy switch 50, and a second engagement structure 84 is arranged inside the first handle 31. When the energy switch 50 is in the release position, the first engagement structure 54 and the second engagement structure 84 are engaged with each other; and when the energy switch 50 is switched between the excitation position and the release position, the first engagement structure 54 moves relative to the second engagement structure 84 so as to be away from or close to the second handle 32. In this way, the energy switch 50 can be movably connected to the first handle 31. Referring to Fig. 2, for example, the prompt tone elastic piece 82 is arranged closer to the main body 10 of the handle assembly than the first engagement structure 54 and the second engagement structure 84; the first engagement structure 54 and the second engagement structure 84 are arranged closer to the main body 10 of the handle assembly than the locking rod 62 and the reset element 63; and the locking rod 62 and the reset element 63 are arranged closer to the main body 10 of the handle assembly than the excitation protrusion 55 and the circuit switch 85. It should be noted that, due to thickly marked reference numerals in Fig. 2, only the engagement slot 53, the pivot 83, the first engagement structure 54 and the second engagement structure 84 are marked in Fig. 3.

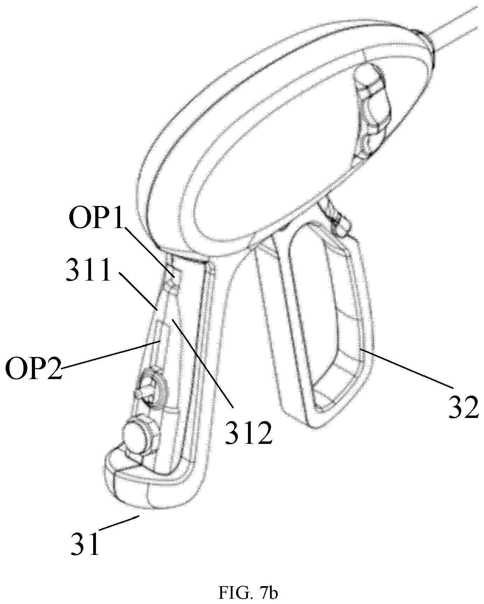

Fig. 7b is a schematic view illustrating an external structure of part A of the surgical instrument shown in Fig. 1, in which the first handle is not provided with an energy switch. Referring to Fig. 2 and Figs. 7a and 7b, the first handle 31 includes a first half-shell 311 and a second half-shell 312, which are connected with each other to define a cavity and a groove located outside the cavity; the pivot 83 and the second engagement structure 84 are located inside the cavity; the bottom of the groove is provided with a first opening OP1 and a second opening OP2. The engagement slot 53 is engaged with the pivot 83 through the first opening OP1, and the first engagement structure 54 is engaged with the second engagement structure 84 through the second opening OP2 so that the energy 50 is connected to the first handle 31 and the groove is covered. For example, the bottom of the groove is also provided with an opening exposing the locking rod 62 and an opening exposing the circuit switch 85, so as to ensure the cooperation between the locking rod 62 and the energy switch 50 and the cooperation between the excitation protrusion 55 and the circuit switch 85. For example, the main body 10 of the handle assembly has a first half-shell and a second half-shell, the first half-shell and the second half-shell of the main body 10 of the handle assembly are integrally formed with the first half-shell 311 and the second half-shell 312 of the first handle 31, respectively, so that the inner space of the main body 10 of the handle assembly communicates with the inner space of the first handle 31.

For example, in the surgical instrument according to the embodiment of the present disclosure, the side of the first handle 31 facing towards the second handle 32 is not provided with any structure deformable under the action of pressing. The second handle 32 moves towards the first handle 31 to allow the end effector 21 to be closed, and to allow the locking assembly to reach the unlocked position, during which the second handle 32 may collide with the first handle 31; since the side of the first handle 31 facing towards the second handle 32 is not provided with any structure deformable under the action of pressing, any spurious triggering of the structure deformable under the action of pressing can be avoided when the second handle 32 collides with the first handle 31. For example, referring to Fig. 1, the side of the second handle 32 facing towards the first handle 31 is flat. For example, referring to Fig. 2, the side of the second handle 32 facing towards the first handle 31 has a collision avoidance protrusion to prevent the second handle 32 from colliding with the first handle 31 in a large area. For example, referring to Fig. 1 and Fig. 2, the surgical instrument according to the embodiment of the present disclosure further includes a rotary structure 13 rotatably connected to the main body 10 of the handle assembly; when the operator rotates the rotary structure 13, the rotary structure 13 drives the first transmission assembly 41 and the end effector 21 as well as the second transmission assembly 42 and the blade 22 to rotate, so that the operator can perform the operation at a proper angle.

What have been described above are merely exemplary embodiments of the present disclosure, but are not used to limit the scope of protection of the present disclosure, which is determined by the appended claims.

## Claims

1. A surgical instrument, comprising:
a handle assembly, comprising a main body, a first handle and a second handle, wherein the first handle is fixedly connected to the main body, and the second handle is movably connected to the main body;
an end effector connected with the main body of the handle assembly;
a first transmission assembly, wherein the second handle, the first transmission assembly and the end effector are configured such that the second handle moves relative to the first handle to drive the first transmission assembly to move, and the first transmission assembly drives the end effector to move; and
an energy switch configured to be switchable between an excitation position and a release position, wherein the energy switch is configured to transmit an energy to the end effector when the energy switch is in the excitation position, and the energy switch is configured not to transmit the energy to the end effector when the energy switch is in the release position, and wherein the energy switch is arranged on the first handle and located at an opposite side of a side of the first handle facing towards the second handle.

2. The surgical instrument according to claim 1, further comprising a locking assembly configured to be switchable between a locked position and an unlocked position, wherein
the locking assembly and the energy switch are configured such that the energy switch is limited in the release position when the locking assembly is in the locked position; and
the locking assembly and the energy switch are configured such that the energy switch is switchable between the excitation position and the release position when the locking assembly is in the unlocked position.

3. The surgical instrument according to claim 2, wherein the locking assembly is connected with the second handle, and the second handle moves relative to the first handle to drive the locking assembly to be switched between the locked position and the unlocked position.

4. The surgical instrument according to claim 3, wherein the second handle, the end effector and the locking assembly are configured such that the second handle moves towards the first handle to drive the locking assembly to move towards the unlocked position and drive the end effector to move towards a closed position through the first transmission assembly, and the second handle moves away from the first handle to drive the locking assembly to move towards the locked position and drive the end effector to move towards an opened position through the first transmission assembly.

5. The surgical instrument according to any one of claims 2-4, wherein the locking assembly comprises a limiting rod, a locking rod and a reset element, wherein
the limiting rod is configured to be switchable between a limiting position and a non-limiting position;
the locking assembly is configured such that when the locking assembly is in the locked position, the limiting rod is in the limiting position and the reset element is in a reset state, and the locking rod is abutted between the energy switch and the limiting rod to limit the energy switch in the release position; and
the locking assembly is configured such that when the locking assembly is in the unlocked position, the limiting rod is in the non-limiting position, an end of the locking rod facing towards the energy switch is abutted against the energy switch, and an end of the locking rod facing towards the limiting rod is not abutted against the limiting rod and becomes a free end, which allows the energy switch to be switchable between the excitation position and the release position; wherein under an action of external force, the energy switch moves from the release position to the excitation position and drives the locking rod to move, and the locking rod drives the reset element to generate a deformation which results in an elastic restoring force; and wherein when the external force is removed, the reset element returns to the reset state under an action of the elastic restoring force and drives the locking rod to move, and the locking rod drives the energy switch to move from the excitation position to the release position.

6. The surgical instrument according to claim 5, wherein
at least a part of the limiting rod is located in the first handle, and is located at a side of the energy switch facing towards the second handle and is spaced apart from the energy switch by an interval; and
the locking rod and the reset element are located in the first handle and are located at the interval between the limiting rod and the energy switch, and extending directions of the locking rod and the reset element intersect with the limiting rod and the energy switch, respectively.

7. The surgical instrument according to claim 5 or 6, wherein
the first handle is provided with an accommodating structure;
the reset element is connected with the locking rod, sleeved onto a part of the locking rod and accommodated, together with the part of the locking rod, in the accommodating structure; wherein the accommodating structure is provided with an opening for the end of the locking rod facing towards the limiting rod to pass therethrough, and a size of the reset element is larger than that of the opening in a direction perpendicular to an extending direction of the locking rod; and
the locking rod is provided with a limiting boss, and the limiting boss is located in the accommodating structure and is closer to the energy switch than the reset element.

8. The surgical instrument according to any one of claims 5-7, wherein
the limiting rod is connected with the second handle, and wherein the second handle moves towards the first handle to drive the limiting rod to move towards the non-limiting position, and the second handle moves away from the first handle to drive the limiting rod to move towards the limiting position.

9. The surgical instrument according to claim 8, wherein
the surgical instrument comprises a first sliding slot at least partially arranged inside the main body of the handle assembly, and the first sliding slot has a first end close to the first handle and a second end away from the first handle;
a part of the second handle located in the main body of the handle assembly is provided with a second sliding slot, wherein the second sliding slot is arranged at a side of the part of the second handle facing towards the first handle, and wherein the second sliding slot has a first end close to the second handle and a second end away from the second handle;
the limiting rod is provided with a first protrusion and a second protrusion which are arranged opposite to each other, wherein the first protrusion is slidably connected in the first sliding slot, and the second protrusion is slidably connected in the second sliding slot;
the first sliding slot is cooperated with the first protrusion and the second sliding slot is cooperated with the second protrusion such that, the second handle moves towards the first handle to allow the second protrusion to slide, in the second sliding slot, from the second end of the second sliding slot towards the first end of the second sliding slot, and allow the first protrusion to slide from the first end of the first sliding slot towards the second end of the first sliding slot, so as to drive the limiting rod to the non-limiting position; and
the first sliding slot is cooperated with the first protrusion and the second sliding slot is cooperated with the second protrusion such that, the second handle moves away from the first handle to allow the second protrusion to slide, in the second sliding slot, from the first end of the second sliding slot towards the second end of the second sliding slot, and allow the first protrusion to slide from the second end of the first sliding slot towards the first end of the first sliding slot, so as to drive the limiting rod to the limiting position.

10. The surgical instrument according to claim 9, wherein
an extending direction of the first sliding slot coincides with an extending direction of the limiting rod; and
an extending direction of the second sliding slot intersects with the extending direction of the first sliding slot.

11. The surgical instrument according to any one of claims 8-10, further comprising a prompt tone elastic piece arranged inside the first handle, wherein
the limiting rod is provided with a third protrusion; and
when the second handle moves towards the first handle to drive the limiting rod to the non-limiting position, the third protrusion triggers the prompt tone elastic piece to generate a prompt tone.

12. The surgical instrument according to any one of claims 1-11, wherein
the energy switch is provided with an excitation protrusion protruding towards an inside of the first handle;
a circuit switch is arranged inside the first handle; and
the energy switch is configured such that, when the energy switch is in the excitation position, the excitation protrusion presses the circuit switch to allow the circuit switch to conduct an energy path from an energy source to the end effector, so that an energy is transmitted from the energy source to the end effector; and the energy switch is further configured such that, when the energy switch is in the release position, the excitation protrusion is spaced apart from the circuit switch to prevent the circuit switch from conducting the energy path from the energy source to the end effector, so that the energy cannot be transmitted from the energy source to the end effector.

13. The surgical instrument according to any one of claims 1-12, wherein
the energy switch is used as a part of a housing of the first handle.

14. The surgical instrument according to claim 13, wherein
the energy switch has a first end and a second end opposite to each other along an extending direction of the energy switch, and the second end is closer to the main body of the handle assembly than the first end;
in a direction from the first end to the second end of the energy switch, the energy switch comprises a first part and a second part, wherein the second part is closer to the main body of the handle assembly than the first part, and wherein the second part is integrally formed with the first part and is bent from the first part towards a direction away from the first handle;
at any position on the first part, a section of the first part perpendicular to the extending direction of the energy switch is in an arched shape, and a height of the arched shape increases along a direction from the second end to the first end of the energy switch.

15. The surgical instrument according to claim 14, wherein
the second part is bent such that an outer contour of an end of the second part close to the main body of the handle assembly matches an outer contour of the main body of the handle assembly.

16. The surgical instrument according to any one of claims 1-15, wherein
the energy switch has a first end and a second end opposite to each other along an extending direction of the energy switch, and the second end is closer to the main body of the handle assembly than the first end;
an engagement slot is arranged at the second end of the energy switch, and a pivot is arranged inside the first handle, wherein the engagement slot is engaged with the pivot and is rotatable around the pivot;
a first engagement structure protruding towards an inside of the first handle is arranged between the first end and the second end of the energy switch, and a second engagement structure is arranged inside the first handle; wherein when the energy switch is in the release position, the first engagement structure and the second engagement structure are engaged with each other; and when the energy switch is switched between the excitation position and the release position, the first engagement structure moves relative to the second engagement structure so as to be away from or close to the second handle.

17. The surgical instrument according to claim 16, wherein the first handle comprises a first half-shell and a second half-shell which are connected with each other to define a cavity and a groove located outside the cavity; the pivot and the second engagement structure are located inside the cavity, and a bottom of the groove is provided with a first opening and a second opening; wherein the engagement slot is engaged with the pivot through the first opening, and the first engagement structure is engaged with the second engagement structure through the second opening, so that the energy switch is connected to the first handle and covers the groove.

18. The surgical instrument according to any one of claims 1-17, wherein a side of the first handle facing towards the second handle is not provided with a structure which is deformable under an action of pressing.

19. The surgical instrument according to any one of claims 1-17, wherein
the surgical instrument comprises a reset mechanism;
the end effector comprises a first arm and a second arm which are openable and closable; and
the second handle, the first transmission assembly, the reset mechanism and the end effector are configured such that, under an action of an external force, the second handle moves towards the first handle to drive the first transmission assembly to move, the reset mechanism generates a deformation resulting in an elastic restoring force, and the first transmission assembly drives the first arm and the second arm of the end effector to be closed; and when the external force is removed, the reset mechanism is reset under an action of the elastic restoring force and drives the first transmission assembly to move, thereby driving the second handle to move away from the first handle and driving the first arm and the second arm of the end effector to be opened.

20. The surgical instrument according to any one of claims 1-17, wherein
the surgical instrument further comprises a blade and a second transmission assembly, wherein the blade is connected to an end of the second transmission assembly facing towards the end effector; and
the handle assembly further comprises a third handle movably connected to the main body of the handle assembly, and wherein the third handle, the second transmission assembly and the blade are configured such that, the third handle moves relative to the first handle to drive the second transmission assembly to move, and the second transmission assembly drives the blade to move towards or away from the end effector.
